# EUROPÄISCHE PATENTSCHRIFT

(11) **EP 3 875 075 B1**
(45) Veröffentlichungstag und Bekanntmachung des Hinweises auf die Patenterteilung: **17.05.2023**
(21) Anmeldenummer: 20160490.7
(22) Anmeldetag: 02.03.2020
(51) Int. Cl.: A61K 9/00, A61K 9/08, A61K 9/10, A61K 47/02, A61K 33/14, A61K 33/42

(54) **ZUBEREITUNG ZUR MAGNETISIERUNG VON NIERENSTEINEN UND NIERENSTEINFRAGMENTEN UND KIT ZUR ENTFERNUNG VON NIERENSTEINEN UND NIERENSTEINFRAGMENTEN**
PREPARATION FOR THE MAGNETISATION OF KIDNEY STONES AND KIDNEY STONE FRAGMENTS AND KIT FOR REMOVING KIDNEY STONES AND KIDNEY STONE FRAGMENTS
PRÉPARATION DE MAGNÉTISATION DES CALCULS RÉNAUX ET DES FRAGMENTS DE CALCULS RÉNAUX ET ENSEMBLE D'ENLÈVEMENT DES CALCULS RÉNAUX ET DES FRAGMENTS DE CALCULS RÉNAUX

(43) Veröffentlichungstag der Anmeldung: 08.09.2021
(73) Patentinhaber: Technische Universität München, 80333 München (DE)
(72) Erfinder: SCHWAMINGER, Sebastian Patrick, Cambridge, MA 02142 (US); SRINIVASAN, Shyam, 80636 München (DE); SOLVIE, Laura Annabelle, 80796 München (DE); TOLOZA, Camilo, 07743 Jena (DE); WENGLER, Michael, 85748 Garching bei München (DE)
(74) Vertreter: Hoefer & Partner Patentanwälte mbB

(56) Entgegenhaltungen:
- EP-A1- 3 090 750
- US-B2- 9 925 311

## Beschreibung

Die vorliegende Erfindung betrifft eine Zubereitung zur Verwendung zur Magnetisierung und zur Entfernung von Nierensteinen oder Nierensteinfragmenten, die ein einfaches Entfernen von Nierensteinen oder Nierensteinfragmenten aus der Niere eines menschlichen Körpers ermöglicht. Des Weiteren betrifft die Erfindung auch ein Kit zur Verwendung zur Magnetisierung und zur Entfernung von Nierensteinen oder Nierensteinfragmenten.

Nierensteine sind kristalline Ablagerungen, die sich in der Niere und auch im Harntrakt eines Säugetiers bilden können. Aktuelle Studien belegen, dass etwa 12% der Weltbevölkerung an Nierensteinen erkrankt, was sehr schmerzhaft ist und zu Folgeschäden führen kann. Oftmals bleibt kein anderer Weg, als die Nierensteine operativ zu entfernen, wobei die Nierensteine vor dem Entfernen je nach Größe zunächst zerkleinert werden, beispielsweise unter Verwendung von Laserpulsen. Die zerkleinerten Nierensteine werden sodann mittels einer Schlinge durch den Harnleiter extrahiert. Nachteilig an diesem Verfahren ist, dass leicht kleinere Nierensteinfragmente übersehen werden können, was zu erneutem Kristallwachstum und Bildung weiterer Nierensteine führt. Aus US 9,925,311 B2 ist ein Kit zur Entfernung von Nierensteinen bekannt, das magnetische oder magnetisierbare Partikel, ein vernetzbares Polymer und ein Vernetzungsmittel umfasst. Hierbei binden die magnetischen oder magnetisierbaren Partikel an die Nierensteine und das vernetzbare Polymer bildet durch Vernetzung mit dem Vernetzungsmittel eine Art Gel, das die durch die magnetischen oder magnetisierbaren Partikel magnetisierten Nierensteine bindet, so dass diese aus dem Körper ausgeschleust werden können. Nachteilig hieran ist, dass die Bereitstellung dieses Kits sehr kostenintensiv ist. Zudem enthält die Zubereitung zum Entfernen der Nierensteine weniger physiologische Komponenten, wie das vernetzende Polymer und das Vernetzungsmittel, was zu Unverträglichkeiten und Schädigungen der Niere führen kann. Auch erfordert die Verwendung des vernetzenden Polymers einen sehr niedrigen pH-Wert um 3,5, was die Handhabung der Zubereitung für den Anwender erschwert.

Ausgehend von diesem Stand der Technik ist es eine Aufgabe der vorliegenden Erfindung eine Zubereitung zur Magnetisierung von Nierensteinen oder Nierensteinfragmenten bereitzustellen, die ein sicheres und einfaches Entfernen von Nierensteinen oder Nierensteinfragmenten aus der Niere eines menschlichen Körpers ermöglicht, Folgeschäden und Folgeerkrankungen vermeidet und für den Anwender gut handhabbar ist. Eine weitere Aufgabe der Erfindung liegt in der Bereitstellung eines Kits, das einfach und sicher in der Anwendung ist und mit dem sowohl Nierensteine als auch Nierensteinfragmente einfach und vollständig entfernt werden können.

Die Aufgabe wird gelöst durch eine Zubereitung zur Verwendung zur Magnetisierung und zur Entfernung von Nierensteinen oder Nierensteinfragmenten, die magnetische Partikel, mindestens ein Salz, ausgewählt aus der Gruppe bestehend aus: Chloriden, Perchloraten und Phosphaten von Lithium (Li), Natrium (Na), Kalium (K), Ammonium (NH₄⁺), Magnesium (Mg) und Calcium (Ca), wie insbesondere LiCl, NaCl, KCl, NH₄Cl, MgCl₂ und CaCl₂, und mindestens ein Lösungsmittel enthält. Hierbei liegt die Zubereitung in Form einer Lösung, einer Suspension oder einer Dispersion vor. Die Verwendung von magnetischen Partikeln ermöglicht bei Anwendung der erfindungsgemäßen Zubereitung eine Magnetisierung der Nierensteine oder Nierensteinfragmente (im Folgenden werden Nierensteine und Nierensteinfragmente unter dem Begriff "Nierensteine" zusammengefasst), so dass diese beispielsweise mittels einer Magnetnadel aus der Niere eines Patienten entfernt werden können. Die mittels der erfindungsgemäßen Zubereitung zu magnetisierenden Nierensteine sind im Einzelnen nicht beschränkt und es kann sich z.B. um Calciumoxalate, Calciumphosphate, Struvitsteine (Ammoniummagnesiumphosphat), Urinsteine oder Cystinsteine handeln.

Unter magnetischen Partikeln werden im Sinne der vorliegenden Erfindung Partikel verstanden, die durch einen Magneten aufgrund magnetischer Wechselwirkung angezogen werden können. Hierbei sind die erfindungsgemäß verwendeten Partikel im Einzelnen nicht beschränkt und können unterschiedliche chemische Zusammensetzungen aufweisen. Bevorzugte magnetische Partikel sind dabei Magnetite, Maghemite und Ferrite, wobei unter Ferriten insbesondere Verbindungen vom Typ XFe₂O₃ mit X = Co, Cu, Ni, Zn, Mn, Ba, Sr und Mg, verstanden werden.

Es wurde überraschend gefunden, dass die Nierensteine und hierunter insbesondere auch sehr kleine Fragmente, vollständig dadurch entfernt werden können, dass mindestens eines der vorstehend genannten Salze oder auch Mischungen von zwei oder mehreren der Salze, zur Anwendung kommt. Das Salz verhindert bzw. unterdrückt hierbei die elektrostatische Abstoßung der magnetischen Partikel, so dass es zu einer verbesserten Partikelaggregation der durch die magnetischen Partikel magnetisierten Nierensteine kommt. Die Nierensteine können somit sehr sicher und einfach vollständig aus einer Niere entfernt werden. Die magnetisierten Nierensteine tendieren somit nicht dazu sich in der Niere zu verteilen, sondern aggregieren vollständig durch Reduktion der repulsiven Wechselwirkungen zwischen den magnetischen Partikeln, ohne dass es chemischer Aggregierungsmittel, wie z.B. vernetzende Polymere, bedarf.

Somit zeichnet sich die erfindungsgemäße Zubereitung durch eine sehr gute Verträglichkeit, einfache und sichere Handhabung sowie eine kostengünstige Zusammensetzung aus. Die Bereitstellung in Form einer Lösung, Suspension oder Dispersion ist aufgrund des verwendeten Salzes auch ohne Konservierungsmittel möglich und erleichtert die Anwendung, da es keiner aufwendigen Bereitstellung der Zubereitung bedarf. Die Zubereitung kann somit bedarfsgerecht bereitgestellt werden.

Die Unteransprüche haben bevorzugte Ausführungsformen und Weiterbildungen der Erfindung zum Inhalt.

Aufgrund der sehr guten Verfügbarkeit sind die magnetischen Partikel vorzugsweise ferromagnetische Partikel und insbesondere ausgewählt aus Eisen, Nickel, Cobalt, AlNiCo, SmCo, Nd₂Fe₁₄B, Ni₈₀Fe₂₀, NiFeCo, Ferriten, Fe₃O₄, gamma-Fe₂O₃, Mischphasen aus Fe₃O₄ und gamma-Fe₂O₃ und Mischungen daraus. Besonders bevorzugt kommen Ferrite, Fe₃O₄, gamma-Fe₂O₃, Mischphasen aus Fe₃O₄ und gamma-Fe₂O₃ und Mischungen aus diesen Eisenoxiden zur Anwendung, da sie über sehr hohe Magnetisierungen in magnetischen Feldern verfügen und damit sehr einfach magnetisch anziehbar sind. Zudem handelt es sich bei diesen magnetischen Partikeln um günstige Partikel, die in beliebigen Partikelgrößen erhältlich sind und sich durch eine gute Verträglichkeit, also keinen nennenswert toxischen Charakter, auszeichnen. Insbesondere können auch Mischungen von metallischen Partikeln, wie z.B. Eisen, Nickel, Cobalt, AlNiCo, SmCo, Nd₂Fe₁₄B, Ni₈₀Fe₂₀ und NiFeCo mit einem oder mehreren der o.g. Metalloxide eingesetzt werden.

Aufgrund der sehr guten Verteilbarkeit in der erfindungsgemäßen Zubereitung beträgt die Primärpartikelgröße der magnetischen Partikel 2 bis 100 nm, insbesondere 4 bis 20 nm und insbesondere 5 bis 15 nm. Je kleiner die Partikelgröße ist, desto besser ist die Verteilbarkeit der magnetischen Partikel in der Zubereitung und somit auch in der Niere eines Patienten. Dies hat jedoch auch den Nachteil, dass eine Aggregation erschwert sein kann. Unter den vorstehend genannten Gesichtspunkten beträgt die Primärpartikelgröße der magnetischen Partikel vorzugsweise 5 bis 15 nm. Die Primärpartikelgröße wird hierbei mittels Transmissionselektronenmikroskopie bestimmt.

Gemäß einer weiteren bevorzugten Weiterbildung weisen die magnetischen Partikel eine spezifische Oberfläche von 80 bis 150 m²/g und insbesondere von 90 bis 120 m²/g auf. Hierdurch wird ein Binden an die Nierensteine verbessert. Die magnetischen Partikel umgeben die Nierensteine und erleichtern damit die Aggregation derselben. Die spezifische Oberfläche wird hierbei mittels Gasadsorptionsisothermen nach dem BET-Verfahren unter Verwendung von Stickstoff bei 77 K bestimmt.

Zur Verbesserung der Langzeitstabilität der Zubereitung und zur Erleichterung der Verteilung der Zubereitung in der Niere eines Patienten unter Anwendung herkömmlicher Injektionsvorrichtungen, beträgt die Konzentration der magnetischen Partikel, bezogen auf die Zubereitung 2 bis 40 g/L und insbesondere 4 bis 20 g/L.

Die repulsive Wechselwirkung zwischen den magnetischen Partikeln kann besonders gut dadurch reduziert und sogar nahezu vollständig unterdrückt werden, dass die Konzentration des Salzes in der Zubereitung vorzugsweise mindestens 100 mmol, insbesondere 100 bis 2000 mmol und insbesondere 200 bis 1000 mmol beträgt. Eine Konzentration im Bereich von 200 bis 1000 millimolar ist dabei aus Gründen der physiologischen Verträglichkeit und auch aus Kostengründen besonders bevorzugt.

Ebenfalls aus Kostengründen sowie aus Gründen einer sehr guten Verträglichkeit, ist das Lösungsmittel vorzugsweise Wasser. Die Verwendung von Alkohol oder Mischungen aus Wasser und Alkohol kann dabei vorteilhaft im Lichte einer verbesserten Aggregation sein, da durch die OH-Gruppen des Alkohols oder der Alkohole ein Binden der Nierensteine an die magnetischen Partikel erleichtert wird. Der Alkohol ist im Einzelnen nicht beschränkt, wobei jedoch Ethanol und Glycerin besonders bevorzugt sind.

Eine weitere vorteilhafte Weiterbildung sieht vor, dass die Zubereitung ferner mindestens einen Zucker und/oder mindestens ein Protein und/oder mindestens einen physiologischen Puffer enthält. Ein Zusatz von Zucker und/oder Protein wirkt sich vorteilhaft dahingehend aus, dass die Viskosität der Zubereitung erhöht wird, so dass die Aggregation von magnetisierten Nierensteinen sowie deren Ausschleusung aus der Niere erleichtert werden.

Aus Gründen der physiologischen Verträglichkeit sowie aus Gründen der Anwendersicherheit liegt der pH-Wert der Zubereitung vorzugsweise in einem Bereich von 5 bis 8 und insbesondere von 6 bis 7. Dies wird insbesondere dadurch möglich, dass die erfindungsgemäße Zubereitung aufgrund ihrer einfachen chemischen Zusammensetzung auf anderweitige, einen sauren oder basischen pH-Wert erfordernde Hilfsstoffe, wie z.B. Tenside, Aggregierungsmittel und dergleichen, verzichten kann.

Insbesondere ist die Zubereitung aus Gründen der Kostenersparnis und physiologischen Verträglichkeit im Wesentlichen frei von filmbildenden und vernetzenden Polymeren. Dies bedeutet, dass der Zubereitung keine vernetzenden Polymere oder auch Vernetzungsmittel für diese Polymere zugesetzt werden.

Ebenfalls erfindungsgemäß wird auch ein Kit zur Entfernung von Nierensteinen oder Nierensteinfragmenten beschrieben. Das Kit umfasst die vorstehend offenbarte Zubereitung sowie eine magnetische oder magnetisierbare Vorrichtung, mittels der die durch die erfindungsgemäße Zubereitung magnetisierten Nierensteine oder Nierensteinfragmente angezogen und aus der Niere eines Patienten entfernt werden können. Besonders geeignete Vorrichtungen sind Magnetnadeln, die ein permanentmagnetisches Material oder ein elektromagnetisches Material umfassen, mit dem die magnetisierten und aggregierten Nierensteine in Wechselwirkung treten und reversibel gebunden werden können. Ein vollständiges Entfernen von Nierensteinen aus der Niere eines Patienten ist damit sehr sicher und einfach möglich.

Weitere Einzelheiten, Merkmale und Vorteile der Erfindung ergeben sich aus der nachfolgenden Beschreibung und den Figuren. Es zeigen:
- Figur 1: eine schematische Ansicht einer Zubereitung gemäß einer Ausführungsform der Erfindung,
- Figur 2: eine schematische Schnittansicht eines mittels der Zubereitung aus Figur 1 behandelten Nierensteins und
- Figur 3: ein Diagramm, veranschaulichend den hydrodynamischen Durchmesser von magnetischen Partikeln in Abhängigkeit der Konzentration von NaCl.

In den Figuren sind nur die wesentlichen Merkmale der vorliegenden Erfindung gezeigt. Alle übrigen Merkmale sind der Übersichtlichkeit halber weggelassen. Zudem beziffern gleiche Bezugszeichen gleiche Komponenten.

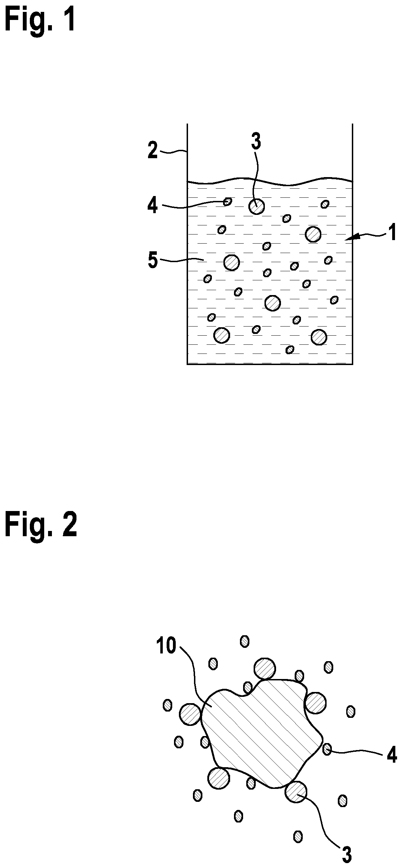

Im Detail zeigt Figur 1 eine in einem Gefäß 2 enthaltene Zubereitung 1 zur Magnetisierung von Nierensteinen oder Nierensteinfragmenten. Die Zubereitung 1 kann beispielsweise in einem chirurgischen Eingriff verwendet werden, bei dem die Zubereitung 1 in die Niere eines Patienten eingeleitet wird.

Die Zubereitung 1 liegt beispielhaft in Form einer Suspension vor und enthält damit ein Lösungsmittel 5 und darin enthaltend magnetische Partikel 3 und ein Salz 4, das aus der Gruppe bestehend aus: Chloriden, Perchloraten und Phosphaten von Li, Na, K, Ammonium, Mg und Ca, wie insbesondere LiCl, NaCl, KCI, NH₄Cl, MgCl₂ und CaCl₂, ausgewählt ist. Als Salz 4 wird vorzugsweise NaCl verwendet, das üblicherweise in dem Lösungsmittel 5, welches vorzugsweise Wasser ist, in gelöster Form vorliegt. Da es sich jedoch bei Figur 1 um eine illustrative Darstellung handelt, ist das Salz 4 in partikulärer Form dargestellt.

Wie Figur 1 zeigt, sind die magnetischen Partikel 3 homogen in der Zubereitung 1 verteilt. Zwischen den magnetischen Partikeln 3 sind lediglich geringe repulsive Wechselwirkungen vorhanden. Die repulsiven Wechselwirkungen werden durch das Salz 4 unterdrückt. Vielmehr werden die magnetischen Partikel 3 durch die elektrostatische Wechselwirkung mit dem Salz 4 räumlich zusammengehalten ohne jedoch zu verklumpen.

Die Zubereitung 1 zeichnet sich durch eine hohe Langzeitstabilität aus, ist sicher in der Anwendung und ermöglicht, wie in Figur 2 schematisch gezeigt, eine Magnetisierung von Nierensteinen und Nierensteinfragmenten. Zudem werden keine weiteren Aggregierungsmittel benötigt, um ein vollständiges Ausschleusen von Nierensteinen und selbst sehr kleinen Nierensteinfragmenten aus einer Niere zu ermöglichen.

Figur 2 zeigt, wie die magnetischen Partikel 3 an den Nierenstein 10 binden. Diese Bindung ist insbesondere kovalent, kann aber auch physikalischer Natur sein. Sobald die magnetischen Partikel 3 mittels der Zubereitung 1 in die Nähe eines Nierensteins 10 gelangen, binden die magnetischen Partikel 3 an die Oberfläche des Nierensteins 10, so dass der Nierenstein 10 "magnetisiert" wird. Eine Aggregation von mehreren, auf dieser Weise magnetisierten Nierensteinen 10 wird durch das Vorhandensein des Salzes 4 erzielt, das die zwischen den magnetischen Partikeln 3 wirkenden repulsiven Wechselwirkungen reduziert oder sogar unterdrückt und damit eine elektrostatische Anziehung der magnetisierten Nierensteine 10 ermöglicht. Somit können die magnetisierten Nierensteine 10 sehr einfach durch eine ebenfalls in die Niere einführbare magnetische Vorrichtung angezogen und aus der Niere entfernt werden.

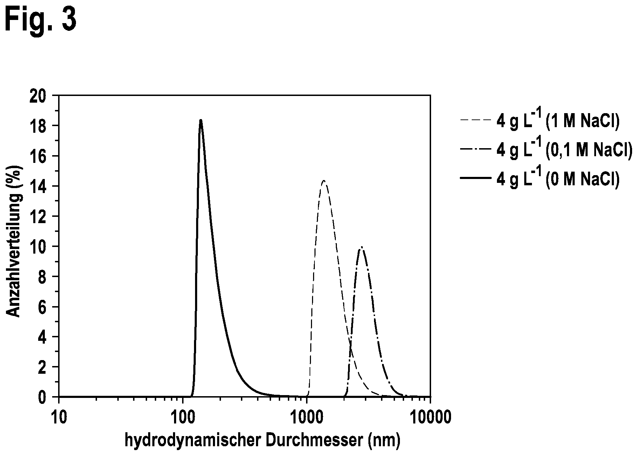

Figur 3 ist ein Diagramm, das den hydrodynamischen Durchmesser von magnetischen Partikeln in Abhängigkeit der Konzentration von NaCl veranschaulicht. Der hydrodynamische Durchmesser ist dabei ein Maß für die Aggregierfähigkeit der magnetischen Partikel. Je größer der hydrodynamische Durchmesser ist, desto besser ist die Aggregierfähigkeit und desto leichter können Nierensteine magnetisiert und nach Aggregation aus einer Niere ausgeschleust werden.

Als magnetische Partikel wurden Partikel von Fe₃O₄ verwendet. Die hier verwendeten Nanopartikel wurden in einer Co-Fällung von Eisen(II)chlorid und Eisen(III)chlorid mit Natronlauge nach dem Massartprozess hergestellt (siehe z.B.: Roth et al. https://doi.org/10.1016/j.jmmm.2014.10.074). Die Partikel wurden mit vollentsalztem Wasser gewaschen und anschließend für die Röntgenbeugung, Stickstoffadsorptionsisothermen nach dem BET Verfahren und Magnetometrie gefriergetrocknet. Die TEM Messungen ergaben einen durchschnittlichen Partikeldurchmesser von 10 nm und das Röntgenbeugungsbild verifizierte Magnetit als Hauptstruktur der Partikel. Für die Transmissionselektronenmikroskopie wurden die Proben in einer Konzentration von ca. 0.01 g/L auf TEM Grids von Quantifoil getropft (10 µL), die zuvor mit Hilfe von Luftplasma für 30 s hydrophilisiert wurden. Die Proben wurden mit einem Kaltluftfön getrocknet und nach einem weiteren Tag in einem JEOL JEM 1400Plus Transmissionselektronenmikroskop vermessen. Das TEM wurde mit Hilfe des Beugungsmusters eines Katalasekristalls kalibriert. Von den Eisenoxidproben wurden jeweils mindestens 5 Aufnahmen gemacht und zur statistischen Analyse der Partikelgröße wurden pro Bild mindestens 30 Partikel mit Hilfe des Bildbearbeitungsprogramms ImageJ vermessen. Mit Röntgenbeugung konnte mit Hilfe des Scherrerverfahrens ein Primärpartikeldurchmesser von 9 nm ermittelt werden. Die magnetometrischen Messungen ergaben eine Sättigungsmagnetisierung von 75 Am²/kg in einem Feld von 50000 Oe. Zudem konnte bei den Partikeln keine Remanenz und daher superparamagnetisches Verhalten festgestellt werden. Die spezifische Oberfläche der Partikel gemäß dem BET Verfahren mit Stickstoff bei 77 K betrug 100 m²/g. Es wurde hierfür ein Gemini VII von Micromeritics genutzt und 9 Punkte zwischen 0,05 und 0,25 p/P₀ vermessen. Das Totvolumen der Probe wurde zuvor mit Helium bei 77 K ermittelt.

Die Partikel wiesen einen hydrodynamischen Durchmesser von 180 nm und einen isoelektrischen Punkt bei pH 6 auf. Diese magnetischen Partikel wurden in einer Konzentration von 4 g/L in Wasser als Lösungsmittel dispergiert.

Auf diese Weise wurden drei Suspensionen hergestellt, die in jeweils ein Gefäß abgefüllt wurden. Gefäß 1 wurde kein NaCl als Salz zugefügt (Konzentration an NaCl: 0 M). Gefäß 2 wurde NaCl zugefügt, so dass die Konzentration an NaCl in der Zubereitung 0,1 M war. Gefäß 2 wurde NaCl zugefügt, so dass die Konzentration an NaCl in der Zubereitung 1 M war.

Das Diagramm zeigt, dass der hydrodynamische Durchmesser mit zunehmender NaCl-Konzentration steigt. Somit steigt auch die Aggregierfähigkeit von mittels der Zubereitung magnetisierten Nierensteinen, da die repulsive Wechselwirkung zwischen den magnetischen Partikeln reduziert ist.

### Bezugszeichenliste:

- 1: Zubereitung
- 2: Gefäß
- 3: magnetische Partikel
- 4: Salz
- 5: Lösungsmittel
- 10: Nierenstein

## Patentansprüche

1. Zubereitung (1) zur Verwendung zur Magnetisierung und zur Entfernung von Nierensteinen (10) oder Nierensteinfragmenten enthaltend:
- magnetische Partikel (3),
- mindestens ein Salz (4), ausgewählt aus der Gruppe bestehend aus: Chloriden, Perchloraten und Phosphaten von Li, Na, K, Ammonium, Mg und Ca und
- mindestens ein Lösungsmittel (5),
wobei die Zubereitung (1) in Form einer Lösung, einer Suspension oder Dispersion vorliegt.

2. Zubereitung (1) zur Verwendung nach Anspruch 1, wobei die magnetischen Partikel (3) ferromagnetische Partikel und insbesondere ausgewählt sind aus Eisen, Nickel, Cobalt, AlNiCo, SmCo, Nd₂Fe₁₄B, Ni₈₀Fe₂₀, NiFeCo, Ferriten, Fe₃O₄, gamma-Fe₂O₃, Mischphasen aus Fe₃O₄ und gamma-Fe₂O₃ und Mischungen daraus.

3. Zubereitung (1) zur Verwendung nach Anspruch 1 oder 2, wobei die magnetischen Partikel (3) eine Primärpartikelgröße von 2 bis 100 nm, insbesondere von 4 bis 20 nm und insbesondere von 5 bis 15 nm aufweisen.

4. Zubereitung (1) zur Verwendung nach einem der vorhergehenden Ansprüche, wobei die magnetischen Partikel (3) eine spezifische Oberfläche von 80 bis 150 m²/g, insbesondere von 90 bis 120 m²/g aufweisen, wobei die spezifische Oberfläche mittels Gasadsorptionsisothermen nach dem BET-Verfahren unter Verwendung von Stickstoff bei 77 K bestimmt wird.

5. Zubereitung (1) zur Verwendung nach einem der vorhergehenden Ansprüche, wobei die Konzentration der magnetischen Partikel (3), bezogen auf die Zubereitung 2 bis 40 g/L und insbesondere 4 bis 20 g/L beträgt.

6. Zubereitung (1) zur Verwendung nach einem der vorhergehenden Ansprüche, wobei die Konzentration des Salzes (4) in der Zubereitung mindestens 100 mmol/L, insbesondere 100 bis 2000 mmol/L und insbesondere 200 bis 1000 mmol/L beträgt.

7. Zubereitung (1) zur Verwendung nach einem der vorhergehenden Ansprüche, wobei das Lösungsmittel (5) ausgewählt ist aus Wasser, Alkohol und Mischungen daraus.

8. Zubereitung (1) zur Verwendung nach einem der vorhergehenden Ansprüche, ferner enthaltend mindestens einen Zucker und/oder mindestens ein Protein und/oder mindestens einen physiologischen Puffer.

9. Zubereitung (1) zur Verwendung nach einem der vorhergehenden Ansprüche, wobei der pH-Wert der Zubereitung (1) in einem Bereich von 5 bis 8, insbesondere von 6 bis 7 liegt.

10. Zubereitung (1) zur Verwendung nach einem der vorhergehenden Ansprüche, wobei die Zubereitung (1) im Wesentlichen frei ist von filmbildenden und vernetzenden Polymeren.

11. Kit zur Verwendung zur Entfernung von Nierensteinen (10) oder Nierensteinfragmenten umfassend die Zubereitung (1) nach einem der vorhergehenden Ansprüche und eine magnetische oder magnetisierbare Vorrichtung, insbesondere eine Magnetnadel, zur Entfernung von durch die Zubereitung magnetisierten Nierensteinen oder Nierensteinfragmenten.

## Claims

1. Preparation (1) for use for the magnetization and removal of kidney stones (10) or kidney stone fragments, comprising:
- magnetic particles (3),
- at least one salt (4) selected from the group consisting of: chlorides, perchlorates and phosphates of Li, Na, K, ammonium, Mg and Ca, and
- at least one solvent (5),
wherein the preparation (1) is in the form of a solution, a suspension or a dispersion.

2. Preparation (1) for use according to claim 1, wherein the magnetic particles (3) are ferromagnetic particles and in particular are selected from iron, nickel, cobalt, AlNiCo, SmCo, Nd₂Fe₁₄B, Ni₈₀Fe₂₀, NiFeCo, ferrites, Fe₃O₄, gamma-Fe₂O₃, mixed phases of Fe₃O₄ and gamma-Fe₂O₃ and mixtures thereof.

3. Preparation (1) for use according to either claim 1 or claim 2, wherein the magnetic particles (3) have a primary particle size of from 2 to 100 nm, in particular from 4 to 20 nm and in particular from 5 to 15 nm.

4. Preparation (1) for use according to any of the preceding claims, wherein the magnetic particles (3) have a specific surface area of 80 to 150 m²/g, in particular from 90 to 120 m²/g, wherein the specific surface area is determined by means of gas adsorption isotherms according to the BET method using nitrogen at 77 K.

5. Preparation (1) for use according to any of the preceding claims, wherein the concentration of the magnetic particles (3), based on the preparation, is 2 to 40 g/L and in particular 4 to 20 g/L.

6. Preparation (1) for use according to any of the preceding claims, wherein the concentration of the salt (4) in the preparation is at least 100 mmol/L, in particular 100 to 2000 mmol/L and in particular 200 to 1000 mmol/L.

7. Preparation (1) for use according to any of the preceding claims, wherein the solvent (5) is selected from water, alcohol and mixtures thereof.

8. Preparation (1) for use according to any of the preceding claims, further comprising at least one sugar and/or at least one protein and/or at least one physiological buffer.

9. Preparation (1) for use according to any of the preceding claims, wherein the pH of the preparation (1) is in a range from 5 to 8, in particular from 6 to 7.

10. Preparation (1) for use according to any of the preceding claims, wherein the preparation (1) is substantially free from film-forming and cross-linking polymers.

11. Kit for use for the removal of kidney stones (10) or kidney stone fragments, comprising the preparation (1) according to any of the preceding claims and a magnetic or magnetizable device, in particular a magnetic needle, for removing kidney stones or kidney fragments magnetized by the preparation.

## Revendications

1. Préparation (1) à utiliser pour la magnétisation et pour l'élimination de calculs rénaux (10) ou fragments de calculs rénaux contenant :
- des particules magnétiques (3),
- au moins un sel (4), choisi dans le groupe constitué de : chlorures, perchlorates et phosphates de Li, Na, K, ammonium, Mg et Ca et
- au moins un solvant (5),
dans laquelle la préparation (1) est présente sous forme d'une solution, d'une suspension ou dispersion.

2. Préparation (1) à utiliser selon la revendication 1, dans laquelle les particules magnétiques (3) sont des particules ferromagnétiques et en particulier choisies parmi le fer, le nickel, le cobalt, l'AINiCo, le SmCo, le Nd₂Fe₁₄B, le Ni₈₀Fe₂₀, le NiFeCo, les ferrites, le Fe₃O₄, le gamma-Fe₂-O₃, les phases mixtes composées de Fe₃O₄ et gamma-Fe₂O₃ et des mélanges de ceux-ci.

3. Préparation (1) à utiliser selon la revendication 1 ou 2, dans laquelle les particules magnétiques (3) présentent une taille de particules primaires de 2 à 100 nm, en particulier de 4 à 20 nm et en particulier de 5 à 15 nm.

4. Préparation (1) à utiliser selon l'une quelconque des revendications précédentes, dans laquelle les particules magnétiques (3) présentent une surface spécifique de 80 à 150 m²/g, en particulier de 90 à 120 m²/g, dans laquelle la surface spécifique est déterminée au moyen d'isothermes d'adsorption de gaz selon le procédé BET au moyen d'azote à 77 K.

5. Préparation (1) à utiliser selon l'une quelconque des revendications précédentes, dans laquelle la concentration des particules magnétiques (3) rapportée à la préparation atteint 2 à 40 g/L et en particulier 4 à 20 g/L.

6. Préparation (1) à utiliser selon l'une quelconque des revendications précédentes, dans laquelle la concentration du sel (4) dans la préparation atteint au moins 100 mmol/L, en particulier 100 à 2000 mmol/L et en particulier 200 à 1000 mmol/L.

7. Préparation (1) à utiliser selon l'une quelconque des revendications précédentes, dans laquelle le solvant (5) est choisi parmi l'eau, l'alcool et les mélanges de ceux-ci.

8. Préparation (1) à utiliser selon l'une quelconque des revendications précédentes, contenant en outre au moins un sucre et/ou au moins une protéine et/ou au moins un tampon physiologique.

9. Préparation (1) à utiliser selon l'une quelconque des revendications précédentes, dans laquelle le pH de la préparation (1) se situe dans une plage de 5 à 8, en particulier de 6 à 7.

10. Préparation (1) à utiliser selon l'une quelconque des revendications précédentes, dans laquelle la préparation (1) est sensiblement exempte de polymères filmogènes et réticulants.

11. Kit à utiliser pour l'élimination de calculs rénaux (10) ou fragments de calculs rénaux comprenant la préparation (1) selon l'une quelconque des revendications précédentes et un dispositif magnétique ou magnétisable, en particulier une aiguille magnétique, pour l'élimination de calculs rénaux ou fragments de calculs rénaux magnétisés par la préparation.
